# EUROPEAN PATENT APPLICATION

(11) **EP 4 039 802 A1**
(43) Date of publication of application: **10.08.2022**
(21) Application number: 20871606.8
(22) Date of filing: 29.09.2020
(51) Int. Cl.: C12N 5/10, C12N 15/10, C12N 15/11, C12Q 1/6851, C12Q 1/686

(54) **QUANTITATIVE PCR METHOD AND KIT THEREFOR**

(30) Priority: 30.09.2019 JP 2019178755; 28.07.2020 JP 2020127716
(71) Applicant: Takeda Pharmaceutical Company Limited, Osaka-shi, Osaka 541-0045 (JP)
(72) Inventor: MATSUMOTO, Shinichi, Fujisawa-shi, Kanagawa 251-0012 (JP); YAMAMOTO, Shunsuke, Fujisawa-shi, Kanagawa 251-0012 (JP); GOTO, Akihiko, Fujisawa-shi, Kanagawa 251-0012 (JP); FUKUDA, Miyuki, Fujisawa-shi, Kanagawa 251-0012 (JP); HIRABAYASHI, Hideki, Fujisawa-shi, Kanagawa 251-0012 (JP)
(74) Representative: Jones, Nicholas Andrew
(86) International application number: PCT/JP2020/036819
(87) International publication number: WO 2021/065877

(57) **Abstract**

The present invention provides an improved quantitative PCR method (qPCR method) for measuring a quantity of a target gene included in a human cell, the method enabling a more accurate analysis of cell kinetics in cell therapy, for example. The qPCR method of the present invention includes a step of adding, to a biological sample including the human cell, a predetermined quantity of exogenous DNA that does not cross over with the target gene as an external standard for correcting a measured quantity of the target gene.

## Description

### Technical Field

The present invention relates to a quantitative polymerase chain reaction (qPCR) method and a kit therefor. More particularly, the present invention relates to a method for correcting a result of quantitative determination of a target gene in a qPCR method and a kit for performing the method.

### Background of Invention

In a therapy in which genetically-engineered cells are administered, for example, in a cell therapy in which T cells expressing a chimeric antigen receptor (CAR) specific for a particular tumor (CAR-T cells) are administered, it is important to evaluate the kinetics of the number of cells (cellular kinetics) *in vivo* after the administration in order to evaluate the therapeutic effect and safety (side effects) and establish an appropriate therapeutic strategy.

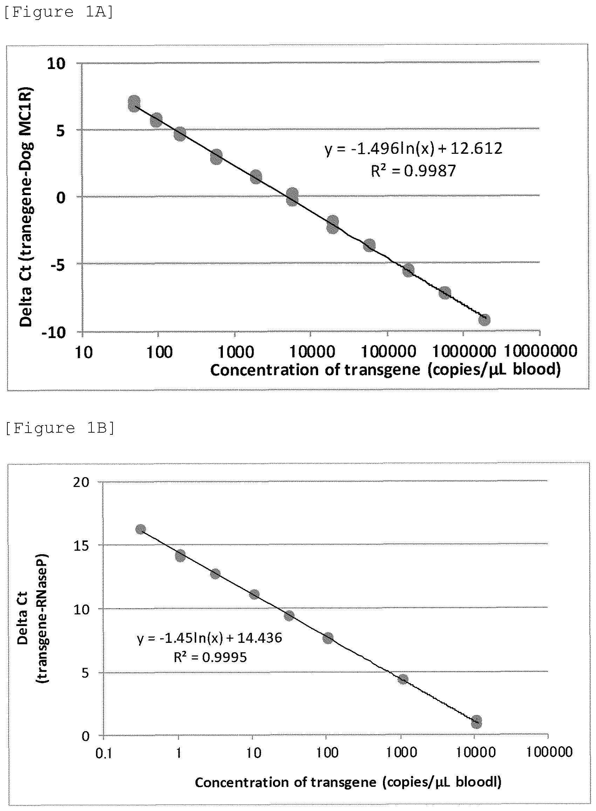

For example, Non Patent Literature 1 discloses the *in vivo* cell kinetics of Tisagenlecleucel (code name CTL019), i.e., a formulation containing CAR-T cells targeting CD19-positive B cells, as administered for the treatment of acute lymphoblastic leukemia (ALL) and chronic lymphocytic leukemia (CLL). The administered CAR-T cells are expected to proliferate *in vivo* and persist number of cells. The number of CAR-T cells in the blood varies in relation to the symptoms of ALL and CLL to be treated, and may also be related to the severity of cytokine release syndrome (CRS) which may be caused as the toxicity of the formulation. Therefore, for such a formulation, the kinetics of the number of CAR-T cells in the blood is considered to be important information for evaluating both the therapeutic effect and safety. In Non Patent Literature 1, the number of copies of a CAR-T gene per µg of genomic DNA (gDNA) (copies/µg gDNA) is used as an index reflecting the number of CAR-T cells. Figure 1 shows that the number of copies rapidly decreases (about 10¹ copies/µg gDNA) in several days from the time of cell transplantation (about 10³ copies/µg gDNA), and then rapidly increases (about 10⁴ to 10⁶ copies/µg gDNA).

### Citation List

### Non Patent Literature

Non Patent Literature 1: Mueller et al., Blood 2017 130:2317-2325; doi: https://doi.org/10.1182/blood-2017-06-786129.

### Summary of Invention

### Technical Problem

Conventionally, a quantitative PCR (qPCR) method targeting a gene specific for cells has been used to quantitatively determine the number of the cells in a biological sample prepared from a specimen such as blood as the number of copies of the gene, as in Non Patent Literature 1, for example. In this case, the number of copies of the target gene is typically standardized by using genomic DNA (gDNA) as an internal standard, for example, a gDNA converted from the quantitative value of a gene whose number of copies in the genome is well known (e.g., the RNaseP gene), and expressed as the number of copies per unit quantity of the gDNA, for example, in units of "copies/µg gDNA".

However, it has not been verified whether a measurement value obtained by such a conventional method for quantitative determination and the cell kinetics based on the measurement value are appropriate as indices for evaluating the efficacy and safety of a cell therapy.

It is an object of the present invention to provide an improved qPCR method that enables a more accurate analysis of cell kinetics in cell therapy, for example.

### Solution to Problem

The present inventors have found that a measurement value of a target gene expressed in units of "copies/µg gDNA" using gDNA as an internal standard in accordance with a conventional typical qPCR method may not be correct enough to be used as information for analyzing the kinetics of CAR-T cells or the like administered in cell therapy, from some viewpoints.

This is because the quantity of gDNA used for standardization is expected to fluctuate relatively widely depending on various factors. For example, in association with the quantity of gDNA in cell therapy, patients typically undergo chemotherapy to deplete lymphocytes in their blood before receiving a transplant of CAR-T cells or the like to enhance the efficacy of the transplant in cell therapy. In that case, the quantity of gDNA in the blood will also temporarily decrease significantly, and then eventually increase as the lymphocytes recover. Also, the CAR-T proliferating will increase the quantity of gDNA. For the reason above, the quantity of gRNA is not necessarily constant among individuals (patients) or among biological samples (samples) from the same individual but collected at different times and may have relatively wide fluctuations or variations. If the quantity of gDNA used as the internal standard fluctuates, the quantitative value of the target gene (copies/µg gDNA), which is expressed relative to the quantity of gDNA, would also fluctuate to a greater extent compared to the case where the quantity of gDNA is assumed to be always constant, and this quantitative value may not be an indicator correctly reflecting the kinetics of proliferation, survival and the like of the CAR-T cells in the blood.

In addition, the fluctuation in the recovery percentage of the target gene may also affect the quantitative value of the number of copies of the target gene. That is, the recovery percentage of a target gene from a DNA extract (the ratio of the number of copies of a target gene contained in an extract obtained from total DNA contained in a biological sample and amplified by qPCR to a true number of copies of the target gene contained in the total DNA) also fluctuates among biological samples, and when the quantity of gDNA is used as an internal standard, the influence of the fluctuation in the recovery percentage of the target gene may not be sufficiently alleviated.

The present inventors have found that by adding exogenous DNA such as dog gDNA to a biological sample and using its quantitative value for standardization, a more correct quantitative value can be obtained with the above various effects on the number of copies of a target gene suppressed and have completed the present invention. In such a qPCR method according to the present invention, the number of copies of the target gene is obtained as a numerical value per unit quantity of biological sample (e.g., blood), for example as a measurement value expressed in units of "copies/µL blood".

In fact, as shown in Comparative Examples 1 to 3 described below, when gDNA was used as an internal standard in conformity with the conventional qPCR method, the quantitative value of the target gene was found to have a relatively low accuracy and a relatively large CV (coefficient of variation). In contrast, in the case of conforming to the present invention using exogenous DNA as an external standard, shown as Examples 1 to 6 described below, the quantitative value of the standard gene had its accuracy and CV significantly improved compared to the conventional method described above, which showed the utility of the quantitative determination method of the present invention. For example, the comparison between Comparative Example 3 and Example 3, in which a sample simulating a biological sample derived from a patient with reduced leukocytes (by removing leukocytes from healthy individual blood) was used, specifically shows the above superiority of the present invention.

Therefore, the present invention provides the following [1] to [6] as a solution to the above problem:
[1] A quantitative PCR method (qPCR method) for measuring a quantity of a target gene included in a human cell, the method comprising a step of:
   adding, to a biological sample including the human cell, a predetermined quantity of exogenous DNA that does not cross over with the target gene as an external standard for correcting a measured quantity of the target gene.
[2] The qPCR method according to item 1, wherein
   the qPCR method is a real-time PCR method, and correction of the measured quantity of the target gene comprises obtaining a difference (ΔCt) between a Ct value measured for the target gene and a Ct value measured for the exogenous DNA and converting the ΔCt into a quantity of the target gene.
[3] The qPCR method according to item 1, wherein correction of the measured quantity of the target gene comprises reflecting a recovery percentage obtained by comparing a measured quantity of the exogenous DNA through the qPCR method with a quantity of the exogenous DNA added to the biological sample.
[4] The qPCR method according to item 1, wherein the qPCR method is a real-time PCR method, and the measured quantity of the target gene is represented by a number of copies per unit quantity of the biological sample.
[5] The qPCR method according to item 1, wherein the human is a human who has genetically-engineered T cells administered and/or suffers from a disease that causes a decrease in leukocytes or has undergone a therapy that causes a decrease in leukocytes.
[6] A kit for qPCR comprising a predetermined quantity of exogenous DNA that does not cross over with a target gene as an external standard for correcting a measured quantity of the target gene.

### Advantageous Effects of Invention

The qPCR method of the present invention, which uses exogenous DNA as an external standard to correct a measurement value, can quantitatively determine a target gene with higher accuracy and CV than the conventional qPCR method using gDNA as an internal standard. Such a qPCR method of the present invention allows a correct evaluation of the kinetics of the number of target gene-containing transplanted cells, for example, in a patient whose gDNA quantity greatly fluctuates due to decreased leukocytes, thereby making it possible to make a highly effective and safe treatment protocol.

### Brief Description of Drawings

[Figure 1A] Figure 1A shows a calibration curve created based on a relationship between the difference (ΔCt; vertical axis) between a Ct value of the target gene and a Ct value of dog genomic DNA (external standard gene) in a calibration curve sample (standard solution) taking immunodeficient mouse blood as a specimen and a concentration of a target gene (horizontal axis) in Example 1.
[Figure 1B] Figure 1B shows a calibration curve created based on a relationship between a Ct value of the target gene (vertical axis) in a calibration curve sample and a concentration of a target gene (horizontal axis) in Comparative Example 1.

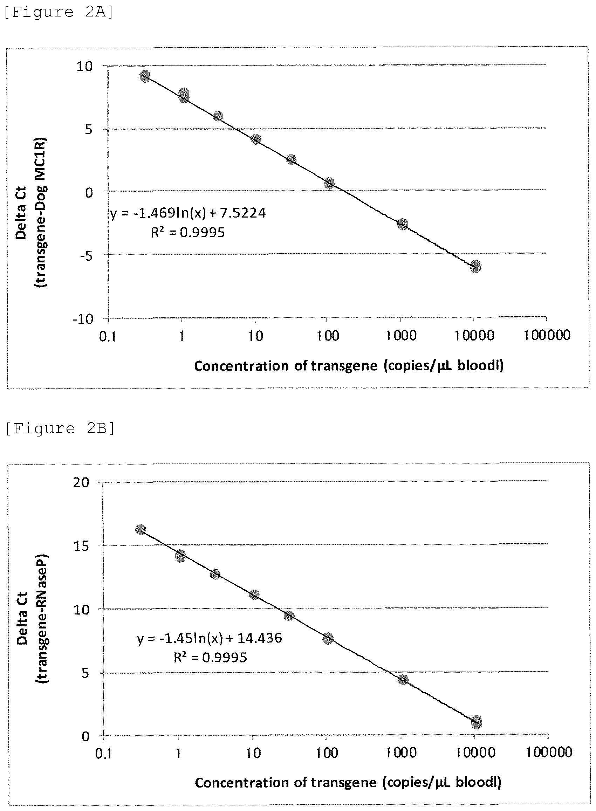

[Figure 2A] Figure 2A shows a calibration curve created based on a relationship between the difference (ΔCt; vertical axis) between a Ct value of the target gene and a Ct value of dog genomic DNA (external standard gene) in a calibration curve sample taking pooled human blood as a specimen and a concentration of a target gene (horizontal axis) in Example 2.
[Figure 2B] Figure 2B shows a calibration curve created based on a relationship between the difference (ΔCt; vertical axis) between a Ct value of the target gene and a Ct value of RNaseP (internal standard gene) in a calibration curve sample taking pooled human blood as a specimen and a concentration of a target gene (horizontal axis) in Comparative Example 2.

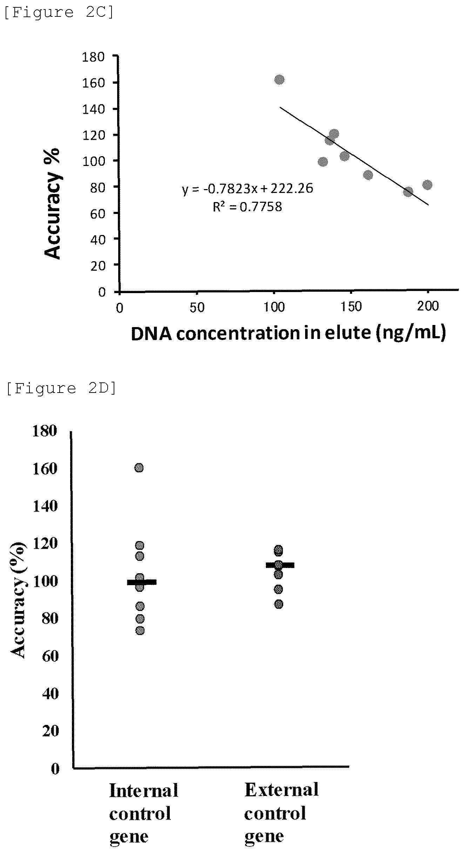

[Figure 2C] Figure 2C is a diagram plotting a correlation between CV (vertical axis, see Table 2B) of the number of copies of a target gene in QC samples prepared from blood samples of individual donors and a DNA concentration (horizontal axis) measured by absorption spectroscopy in Comparative Example 2.
[Figure 2D] Figure 2D is a scatter diagram plotting the accuracy of the number of copies of a target gene in QC samples prepared from blood of individual donors in Example 2 ("External control gene" on the left) and Comparative Example 2 ("Internal control gene" on the right).

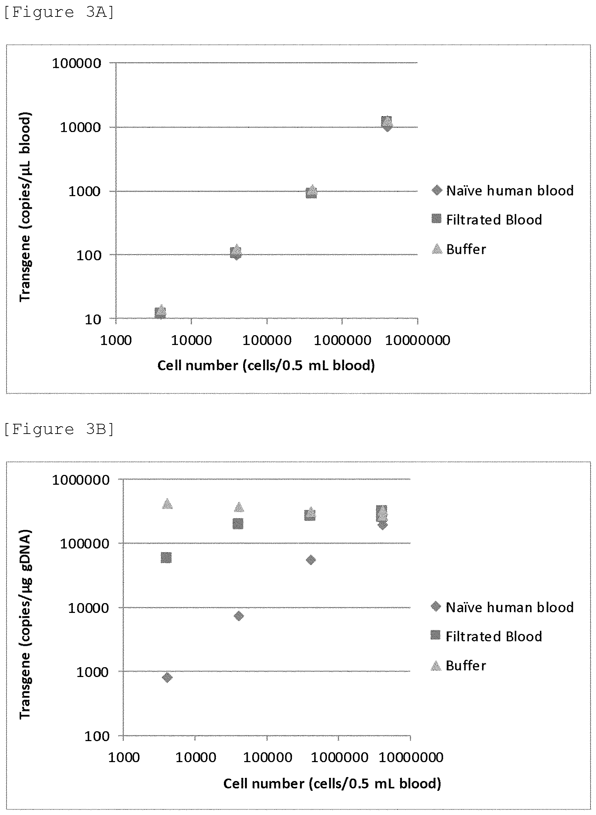

[Figure 3A] Figure 3A shows a relationship between a concentration of a target gene (vertical axis) quantitatively determined from a calibration curve created using dog genomic DNA (external standard gene) and the number of cells added (horizontal axis) for samples of human blood (marked with ◆), leukocyte-removed human blood (marked with ■), and a buffer solution (marked with ▲) each having genetically-engineered human T cells including the target gene added in Example 3.
[Figure 3B] Figure 3B shows a relationship between a concentration of a target gene (vertical axis) quantitatively determined from a calibration curve created using RNaseP (internal standard gene) and the number of cells added (horizontal axis) for samples of human blood (marked with ◆), leukocyte-removed human blood (marked with ■), and a buffer solution (marked with ▲) each having genetically-engineered human T cells including the target gene added in Comparative Example 3.

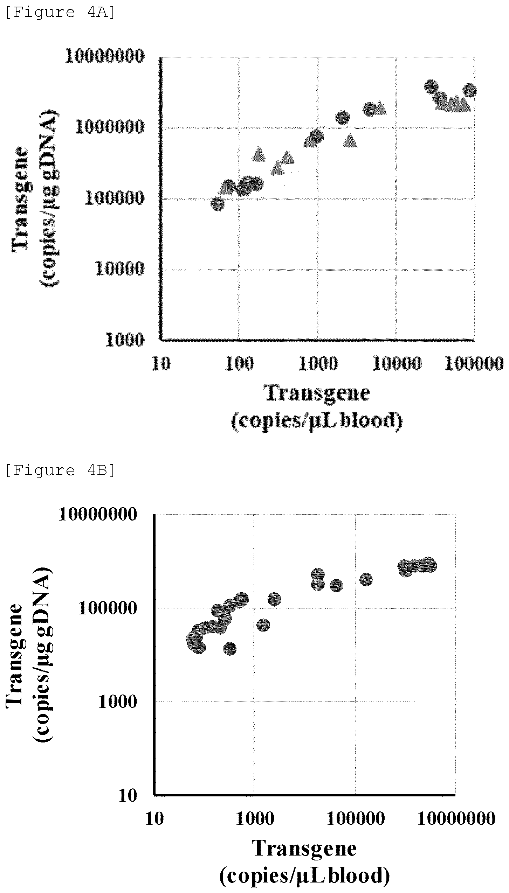

[Figure 4A] Figure 4A is a graph showing a relationship between the result expressed in copies/µL blood by a quantitative determination method using an external standard gene (dog genomic DNA) according to the present invention (horizontal axis) and the result expressed in copies/µg gDNA by a quantitative determination method using an internal standard gene (RNaseP) (vertical axis) for target gene 1 in a blood specimen sampled from a xenograft mouse having first genetically-engineered human T cells administered in Example 6. Plots marked with a circle (•) indicates a case with administration at a dose of 5 × 10⁶ CAR+ cells/animal, and plots marked with a triangle (▲) indicates a case with administration at a dose of 10 × 10⁶ CAR+ cells/animal.
[Figure 4B] Figure 4B is a graph showing a relationship between the result expressed in copies/µL blood by a quantitative determination method using an external standard gene according to the present invention (horizontal axis) and the result expressed in copies/µg gDNA by a quantitative determination method using an internal standard gene (vertical axis) for target gene 2 in a blood specimen sampled from a xenograft mouse having second genetically-engineered human T cells administered in Example 6.

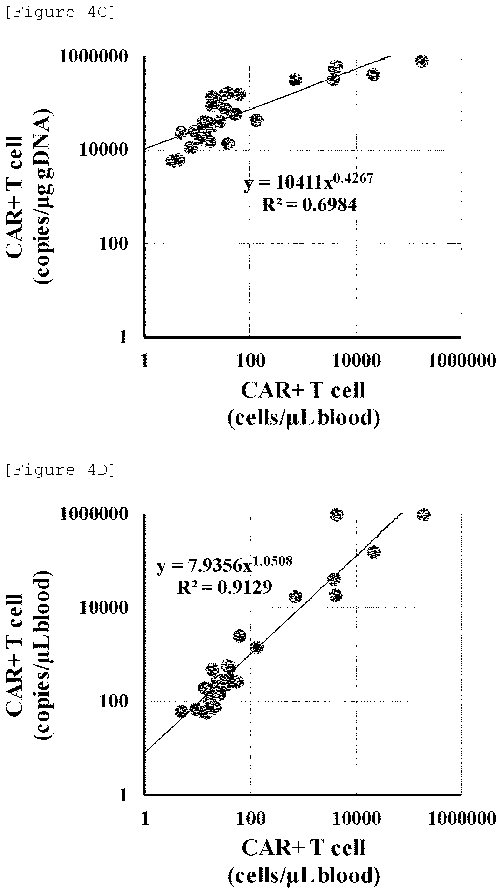

[Figure 4C] Figure 4C is a graph showing a correlation between the number of the second genetically-engineered human T cells in a blood sample for the second genetically-engineered human T cells (horizontal axis, cells/µL blood, measured by flow cytometry) and the level of introduction of the target gene 2 (vertical axis, copies/µg gDNA, measured by the quantitative determination method of the present invention using an internal standard gene) in Example 6.
[Figure 4D] Figure 4D is a graph showing a correlation between the number of the second genetically-engineered human T cells in a blood sample for the second genetically-engineered human T cells (horizontal axis, cells/µL blood, measured by flow cytometry) and the level of introduction of the target gene 2 (vertical axis, copies/µL blood, measured by the quantitative determination method of the present invention using an external standard gene) in Example 6.

### Description of Embodiments

### -qPCR method-

A qPCR method according to the present invention is for measuring a quantity of a target gene included in a human cell and comprises a step of:
adding, to a biological sample including the human cell, a predetermined quantity of exogenous DNA that does not cross over with the target gene as an external standard for correcting a measured quantity of the target gene.

### [Target gene]

The term "target gene" in the present invention is not particularly limited and can be selected from genes contained in human cells that are subjects for quantitative determination by a typical qPCR method.

In one preferred embodiment of the present invention, the target gene is a gene included in a "genetically-engineered T cell". The "T cell" encompasses αβ cells, γδT cells, CD8+ T cells, CD4+ T cells, tumor infiltrating T cells, memory T cells, naive T cells, NKT cells, etc. The term "genetic engineering" encompasses genetic engineering by introduction of TCR (T cell receptor) and the like as well as genetic engineering by introduction of CAR. Representative examples of the genetically-engineered T cell include CAR-T cells and TCR-T cells.

The target gene may be a gene included in "genetically-engineered immunocytes" other than the genetically-engineered T cell, for example, immunocytes modified by introducing a gene such as CAR into NK cells, monocytes, macrophages, dendritic cells, or the like.

The target gene may be, for example, a gene included in an expression vector that has been introduced into a host cell such as a T cell in order to produce a genetically-engineered cell such as a CAR-T cell. The expression vector may be linear or cyclic and may be a non-viral vector such as a plasmid, a viral vector, or a transposon-based vector. Examples of the viral vector include a retroviral vector, a lentiviral vector, an adenoviral vector, and an adeno-associated virus vector. Preferred examples of the retroviral vector include pMSGV vector (Tamada k et al., ClinCancer Res 18:6436-6445 (2002)) and pMSCV vector (produced by Takara Bio Inc.). When a retroviral vector is used, a gene included in the vector is incorporated into the genome of a host cell such as a T cell, and thus the gene can be stably expressed for a long period of time.

For example, when a CAR-T cell is produced as the genetically-engineered T cell, a CAR-expressing gene is introduced into a T cell serving as a host cell; the CAR-expressing gene is basically constructed of peptides for each of the following sites being linked via spacers as necessary: (i) a single-chain antibody that recognizes a cell surface antigen of a target cell for attack such as a cancer cell, (ii) a transmembrane domain, and (iii) a signal transduction domain that induces activation of the T cell. If necessary, the CAR-expressing gene may be used in combination with a suicide gene (a gene for enabling to control the number of genetically-engineered T cells *in vivo* by administering a drug that activates the function of the suicide gene according to the course of treatment after the genetically-engineered T cells have been administered, for example, when the cancer cell to be attacked have disappeared), a cytokine/chemokine gene, a linker for linking genes (P2A, F2A, T2A, E2A sequence, or the like), or the like. When a gene group for producing a CAR-T cell that includes such a CAR-expressing gene, other genes, a linker, and the like is used, the target gene can be a portion (gene or a part thereof) that includes at least a base sequence that allows the target gene to be identified as a gene included in the gene group for producing a CAR-T cell. Similarly, when a TCR-T cell is produced as the genetically-engineered T cell, the target gene can be a portion (gene or a part thereof) that includes at least a base sequence that allows the target gene to be identified as the gene group for producing a TCR-T cell, wherein the foregoing gene group is selected from among the gene group for producing a CAR-T cell including a suicide gene, a cytokine/chemokine gene, a linker for linking genes, etc., if necessary, in addition to a TCR gene.

### [Exogenous DNA]

The exogenous DNA in the present invention is DNA used as an external standard for correcting a quantitative value of a target gene in the qPCR method and has a base sequence that does not cross over with the target gene, namely, a base sequence that is not amplified by a primer for the target gene (a primer for such a target sequence can be designed). Conversely, it is required that the exogenous DNA also be a base sequence that does not cross over with the target gene or other genes in the target cell or biological sample (a primer for such exogenous DNA can be designed). It is desirable that the exogenous DNA includes a base sequence (gene or the like) that is not possessed by humans.

The exogenous DNA is not particularly limited as long as it is DNA having a base sequence with the above-described characteristics. Representative examples of the exogenous DNA include genomic DNA of non-human mammals, preferably non-primate mammals, such as cows, pigs, goats, sheep, dogs, cats, rabbits, and rodents (mice, rats, etc.). It is also possible to use an artificial nucleic acid including an artificially designed and synthesized sequence as the exogenous DNA (an exogenous nucleic acid when the artificial nucleic acid includes a nucleic acid other than DNA, such as LNA) .

The exogenous DNA may be DNA isolated in advance so that the quantity of addition can be easily adjusted or may be DNA contained in cells of a mammal other than humans (the quantity of addition will be adjusted as the number of cells). The exogenous DNA contained in cells may be extracted from the cells together with the target gene contained in the cells.

The quantity of the exogenous DNA added to a biological sample is not particularly limited and can be appropriately adjusted. For example, in a typical embodiment of the present invention as described below, when the difference (ΔCt) between a Ct value of the target gene and a Ct value of the exogenous DNA is calculated, the quantity of addition may be such that the Ct value can be obtained at least for the exogenous DNA (the quantity of the amplification product of the exogenous DNA will reach a threshold in a predetermined number of PCR cycles or below).

### [Biological sample]

For the "biological sample" in the present invention, the same biological sample as that for quantitatively determining a target gene contained in human cells by a typical qPCR method can be used. Examples of such a biological sample include samples prepared from specimens sampled from living bodies such as blood, spinal fluid, bone marrow, tumors (primary tumor, metastatic tumor, cancerous ascites, and the like), and other specimens that may have cells containing the target gene.

In one embodiment of the present invention, the biological sample is prepared from a specimen sampled from a human who has the genetically-engineered T cells administered and/or suffers from a disease that causes a decrease in leukocytes or has received a therapy therefor. Patients having the genetically-engineered T cells administered are particularly amenable to the analysis of the kinetics of the number of these cells after the administration based on an improved quantitative value than before by the qPCR method of the present invention. In addition, patients with leukopenia (neutropenia, lymphopenia, monocytopenia) or patients who have undergone chemotherapy to deplete leukocytes (lymphocytes) for cell therapy have a significant decrease in the quantity of gDNA, which is used as an internal standard in the conventional technology, and such a decrease significantly affects the quantitative value of a target gene; thus, the application of the qPCR method of the present invention is particularly preferable for these patients.

### [Procedure]

The quantitative PCR method (qPCR method) refers to various PCR methods that can quantitatively measure the number of copies of a gene in the biological sample. Such qPCR methods are well known to and commonly used by those skilled in the art, and their basic and preferred technical matters can be applied to the present invention.

One of the representative embodiments of the qPCR method is a "real-time PCR method". In the real-time PCR method, the fluorescence (signal) emitted according to an increase in an amplified product due to PCR is monitored in real time, and the number of cycles until the amplified product (signal) reaches a certain level is measured, and the number of copies of a target gene in an original biological sample is estimated by referring to the calibration curve created using a standard sample. Specific examples of the real-time PCR method include a fluorescent probe method using a fluorescently labeled probe (e.g., a TaqMan method, a molecular beacon method, a cycling probe method), and an intercalater method using a reagent that emits fluorescence by binding to double-stranded DNA; either method can be used in the present invention.

Another representative embodiment of the qPCR method is a digital droplet PCR method ("ddPCR method"). In the ddPCR method, PCR is performed after limiting dilution of a biological sample so that each microcompartment (well) contains 0 or 1 (or more) target gene, and the absolute number of copies of the target gene in the biological sample is estimated from the proportion of the number of microcompartments with negative amplification signals (not containing the target gene) to the total number of microcompartments. In this estimation, the Poisson distribution is used for correction regarding the fact that there may be a microcompartment containing a plurality of target genes. In the ddPCR method, it is not necessary to create a calibration curve. The amplification signal in the ddPCR method can be based on a fluorescent probe method or the like similar to that exemplified for the real-time PCR method.

Basic reagents, kits, systems, etc. for implementing the qPCR method (real-time PCR method, ddPCR method, etc.) are also commercially available and can be used in the present invention as well. The base sequence and base length of primers (forward primer and reverse primer) respectively for the target gene and the exogenous DNA, which are necessary to implement the qPCR method of the present invention, and of probes used when employing the fluorescent probe method, can be appropriately designed by those skilled in the art to specifically amplify the target gene and the exogenous DNA (so that they do not cross over with other genes). The probes and primers may have a sequence that is completely complementary to the target sequence, a sequence that contains a partial mismatch, or a base sequence having homology within a range that allows it to hybridize to these sequences at high stringency (under stringent hybridization conditions).

A fluorescent substance used when the fluorescent probe method is employed and a quencher that forms a FRET pair with this substance are not particularly limited and can be appropriately selected by those skilled in the art.

The qPCR method of the present invention comprises a step of adding, to a biological sample, a predetermined quantity of exogenous DNA that does not cross over with a target gene as an external standard for correcting a measured quantity of the target gene, and the rest of steps such as steps of preparing a biological sample from a specimen, extracting DNA from human cells or the like, and amplifying nucleic acid by PCR (thermal denaturation, annealing of primers, hybridization of a probe, extension reaction, etc.) can be the same as those of a typical qPCR method.

In the real-time PCR method, which is one representative example of the qPCR method, the number of cycles (Ct value) of the PCR reaction required for the quantity of a reaction product (synthesized and amplified DNA) to reach a certain quantity, that is, for an amplification curve drawn by the intensity of the fluorescence emitted from the fluorescent substance to cross over with a predetermined threshold, is measured and used as a measurement value reflecting the number of copies (concentration) of the target gene contained in the initial biological sample. The larger the Ct value, the lower the number of copies (concentration) of the original DNA. Since DNA increases exponentially in PCR, the difference between two Ct values is related to a logarithmic difference, i.e., the ratio (index) of the number of copies of the original DNA.

In a typical conventional real-time PCR method, the measurement value (Ct value) and the final measured quantity (number of copies) of a target gene have been corrected by the Ct value for gDNA used as an internal standard. That is, the following operations [B1] to [B3] have been performed:
[B1] Measure the Ct value for gDNA used as an internal standard and calculate the difference (ΔCt (i)) between the Ct value of the target gene and the Ct value of the internal standard, thereby correcting the original measurement value (Ct) ;
[B2] Meanwhile also measure the Ct values for a plurality of standard samples with known numbers of copies (concentrations) of the target gene and calculate the differences (ΔCt (i_s)) from the Ct value of the internal standard to create a calibration curve based on the relationship between the plurality of ΔCt (i_s) and their respective corresponding known numbers of copies of the target gene;
[B3] By referring to the calibration curve, convert the corrected measurement value (ΔCt (i)) to obtain a corrected measured quantity for the number of copies of the target gene contained in the initial biological sample. The final measured quantity is expressed, for example, in units of "copies/µg gDNA".

In contrast, in a typical real-time PCR method of the present invention, the measurement value (Ct value) and the final measured quantity (number of copies) of a target gene are corrected by the Ct value for exogenous DNA used as an external standard. That is, the following operations [A1] to [A3] have been performed:
[A1] Measure the Ct value for exogenous DNA used as an external standard and calculate the difference (ΔCt (o)) between the Ct value of the target gene and the Ct value of the external standard, thereby correcting the original measurement value (Ct);
[A2] Meanwhile also measure the Ct values for a plurality of standard samples with known numbers of copies (concentrations) of the target gene and calculate the differences (ΔCt (o_s)) from the Ct value of the external standard to create a calibration curve based on the relationship between the plurality of ΔCt (o_s) and their respective corresponding known numbers of copies of the target gene;
[A3] By referring to the calibration curve, convert the corrected measurement value (ΔCt (o)) to obtain a corrected measured quantity for the number of copies of the target gene contained in the initial biological sample. The final measured quantity can be expressed in the number of copies per unit quantity (weight) of the biological sample, for example, in units of "copies/µg blood".

However, the way of correcting the measured quantity of the target gene using the exogenous DNA in the qPCR method according to the present invention is not limited to the correction regarding ΔCt in the real-time PCR method as described above. If the number of copies of the target gene in the biological sample can be quantitatively determined more correctly than by using the quantitative value itself measured for the target gene, it is possible to use exogenous DNA as an external standard for the correction also in other methods, for example, in the ddPCR method.

For example, for the exogenous DNA, it is conceivable to compare the number of copies of the exogenous DNA actually added to the biological sample with the result of measuring the Ct value of the exogenous DNA in the real-time PCR method and converting it into the number of copies in the biological sample based on the calibration curve or with the result of directly measuring the number of copies in the biological sample in the ddPCR method to confirm whether they match, in other words, to determine the "recovery percentage" (%, (measured quantity/ actual quantity of addition) × 100). If necessary, the measured quantity of the target gene measured by the real-time PCR method, ddPCR method or the like can be further corrected (multiplied by 100/recovery percentage) according to how much the recovery percentage deviates from 100%. Such a further correction can also be performed in combination with the correction regarding ΔCt in the real-time PCR method described above.

### -Kit-

A kit for qPCR according to the present invention comprises a predetermined quantity of exogenous DNA that does not cross over with a target gene as an external standard for correcting a measured quantity of the target gene, and the rest of the components (e.g., a DNA polymerase having a 5' to 3' exonuclease activity, other reagents, and instruments necessary for implementing the qPCR method) may include the same as those of a kit for conventional qPCR. Primers and probes may be separately prepared according to the target gene and the exogenous gene.

### Examples

A probe, forward primer, and reverse primer for a dog genomic DNA-specific gene (MC1R); a probe, forward primer, and reverse primer for target gene 1 (transgene 1 for production of CAR-T cells); and a probe, forward primer, and reverse primer for target gene 2 (transgene 2 for production of CAR-T cells) used in the following Examples and Comparative Examples respectively have the following base sequences. It was confirmed that the probe, forward primer, and reverse primer for the target gene 1 and the probe, forward primer, and reverse primer for the dog genomic DNA-specific gene (MC1R) have base sequences that do not cross each other, and that the probe, forward primer and reverse primer for target gene 2 and the probe, forward primer and reverse primer for the dog genomic DNA-specific gene (MC1R) have base sequences that do not cross each other.
Probe for MC1R (SEQ ID NO: 1)
   5'-GCCTTGGCTGCGCAGGCTGCTGTGGTGCAG-3'
Forward primer for MC1R (SEQ ID NO: 2)
   5'-CGCCCATGTATTACTTCATCTGTTGCC-3'
Reverse primer for MC1R (SEQ ID NO: 3)
   5'-CACGGCGATGGCGCCCAGGAA-3'
Probe for target gene 1 (SEQ ID NO: 4)
   5'-CGACTACAGGGCCTACT-3'
Forward primer for target gene 1 (SEQ ID NO: 5)
   5'-GGAGCTGAGGTCCCTGAGAAG-3'
Reverse primer for target gene 1 (SEQ ID NO: 6)
   5'-CCTGGCCCCAGTAGTCGAA-3'
Probe for target gene 2 (SEQ ID NO: 7)
   5'-CTGAGGAGCGAGGATG-3'
Forward primer for target gene 2 (SEQ ID NO: 8)
   5'-CCGGAGTGCTGCTGATCAG-3'
Reverse primer for target gene 2 (SEQ ID NO: 9)
   5'-GCTGTGCCAGATCATGCAGTA-3'

### [Example 1/Comparative Example 1] Examination of utility of an external standard gene in quantitatively determining a blood concentration of a target gene (the number of copies per unit quantity of a biological sample)

In the description of this Example and this Comparative Example, a "target gene" refers to "target gene 1". Samples were prepared by taking 30 µL of immunodeficient mouse blood as a specimen and adding plasmids containing a target gene thereto at 50, 100, 200, 600, 2,000, 6,000, 20,000, 60,000, 200,000, 600,000, and 2,000,000 copies/µL blood and used as calibration curve samples (standard solutions). Another samples were prepared by adding plasmids containing the target gene at 50, 100, 200, 600, 4,000, 40,000, 400,000, and 2,000,000 copies/µL blood and used as quality control samples (QC samples). To each of all these samples, 1,200 ng of dog genomic DNA (Zyagen) was added as an external standard gene. Genomic DNA was extracted from each of these samples using a commercially available DNA extraction reagent (QIAamp DNA Blood Midi Kits, QIAGEN), and then, qPCR quantification was performed using probes, forward primers and reverse primers respectively for the target gene and the dog genomic DNA-specific gene (MC1R). The qPCR was performed using QuantStudio 7 Flex real-time PCR system (Thermo Fisher Scientific Inc.) for 40 cycles under conditions of 95°C for 10 min/(95°C for 15 seconds and 60°C for 1 minute) as one cycle.

A calibration curve was created using the difference (Delta Ct) between the number of PCR cycles (Ct value) at the time when the target gene amplification curve with each concentration (the number of copies) of the calibration curve samples for the target gene crosses over with a threshold and the Ct value for the amplification curve of the dog genomic DNA in each sample, and the concentration of each calibration curve sample (Figure 1A). Linearity of the calibration curve was observed in a copy number range for the target gene from 50 to 2,000,000 copies/µL blood.

In addition, as a result of creating a calibration curve from the Ct value of the target gene and the concentration of each calibration curve sample, linearity of the calibration curve was observed in a copy number range for the target gene from 50 to 2,000,000 copies/µL blood (Figure 1B).

The calibration curves of the Delta Ct and the Ct value were used to calculate the numbers of copies of the target gene in the QC samples, and the results are shown in Tables 1A and B, respectively. The values (Observed) calculated by the calibration curve of Delta Ct had a mean accuracy (Accuracy, Mean) of 83.0 to 108.1% and a CV (coefficient of variation) of 2.4 to 17.5%, which were good, whereas the values calculated by the calibration curve of Ct had a mean accuracy of 46.9 to 92.7% and a CV of 5.0 to 26.6%; and the method using the calibration curve of Ct (Comparative Example 1) was less quantitative as compared with the results from the calibration curve of Delta Ct (Example 1) using the external standard gene. These results suggested that using the external standard gene to correct the numbers of copies of the target gene among the samples is useful in quantitatively determining the number of copies per unit quantity of a biological sample.

**[Table 1A]**

| Nominal (copies/µL Blood) | Observed (copies/µL Blood) | Accuracy (%) | Mean | C.V. (%) |
|---|---|---|---|---|
| 2000000 | 1837898 | 91.9 | 87.2 | 4.7 |
| 2000000 | 1716677 | 85.8 | | |
| 2000000 | 1712160 | 85.6 | | |
| 2000000 | 1637850 | 81.9 | | |
| 2000000 | 1815645 | 90.8 | | |
| 400000 | 327737 | 81.9 | 83.0 | 7.9 |
| 400000 | 297474 | 74.4 | | |
| 400000 | 334024 | 83.5 | | |
| 400000 | 329580 | 82.4 | | |
| 400000 | 370692 | 92.7 | | |
| 40000 | 35388 | 88.5 | 86.4 | 2.4 |
| 40000 | 35446 | 88.6 | | |
| 40000 | 33644 | 84.1 | | |
| 40000 | 34405 | 86.0 | | |
| 40000 | 33856 | 84.6 | | |
| 4000 | 3636 | 90.9 | 87.7 | 3.3 |
| 4000 | 3336 | 83.4 | | |
| 4000 | 3591 | 89.8 | | |
| 4000 | 3471 | 86.8 | | |
| 4000 | 3509 | 87.7 | | |
| 600 | 608 | 101.3 | 105.6 | 7.2 |
| 600 | 627 | 104.5 | | |
| 600 | 703 | 117.2 | | |
| 600 | 647 | 107.8 | | |
| 600 | 582 | 96.9 | | |
| 200 | 198 | 98.9 | 91.9 | 10.2 |
| 200 | 161 | 80.3 | | |
| 200 | 185 | 92.4 | | |
| 200 | 170 | 84.9 | | |
| 200 | 206 | 102.8 | | |
| 100 | 91 | 91.0 | 108.1 | 10.2 |
| 100 | 107 | 107.1 | | |
| 100 | 117 | 116.5 | | |
| 100 | 107 | 106.6 | | |
| 100 | 119 | 119.2 | | |
| 50 | 60 | 119.9 | 103.6 | 17.5 |
| 50 | 43 | 86.3 | | |
| 50 | 49 | 97.9 | | |
| 50 | 44 | 88.3 | | |
| 50 | 63 | 125.7 | | |

**[Table 1B]**

| Nominal (copies/µL Blood) | Observed (copies/µL Blood) | Accuracy (%) | Mean | C.V. (%) |
|---|---|---|---|---|
| 2000000 | 1084625 | 54.2 | 69.6 | 14.3 |
| 2000000 | 1500596 | 75.0 | | |
| 2000000 | 1617055 | 80.9 | | |
| 2000000 | 1365305 | 68.3 | | |
| 2000000 | 1394588 | 69.7 | | |
| 400000 | 272936 | 68.2 | 78.1 | 10.0 |
| 400000 | 354987 | 88.7 | | |
| 400000 | 313298 | 78.3 | | |
| 400000 | 294181 | 73.5 | | |
| 400000 | 325891 | 81.5 | | |
| 40000 | 32609 | 81.5 | 79.3 | 21.6 |
| 40000 | 40853 | 102.1 | | |
| 40000 | 35082 | 87.7 | | |
| 40000 | 26104 | 65.3 | | |
| 40000 | 23962 | 59.9 | | |
| 4000 | 3510 | 87.8 | 84.2 | 5.0 |
| 4000 | 3561 | 89.0 | | |
| 4000 | 3299 | 82.5 | | |
| 4000 | 3156 | 78.9 | | |
| 4000 | 3305 | 82.6 | | |
| 600 | 581 | 96.8 | 92.7 | 18.1 |
| 600 | 583 | 97.2 | | |
| 600 | 626 | 104.3 | | |
| 600 | 613 | 102.1 | | |
| 600 | 379 | 63.2 | | |
| 200 | 107 | 53.3 | 46.9 | 11.8 |
| 200 | 79 | 39.5 | | |
| 200 | 102 | 51.1 | | |
| 200 | 88 | 43.8 | | |
| 200 | 93 | 46.7 | | |
| 100 | 51 | 51.1 | 64.6 | 22.6 |
| 100 | 62 | 62.3 | | |
| 100 | 89 | 89.4 | | |
| 100 | 58 | 57.8 | | |
| 100 | 62 | 62.2 | | |
| 50 | 38 | 76.0 | 59.4 | 26.6 |
| 50 | 17 | 33.6 | | |
| 50 | 32 | 64.5 | | |
| 50 | 33 | 65.1 | | |
| 50 | 29 | 57.8 | | |

### [Example 2] Quantitative determination of a concentration of a target gene (the number of copies per unit quantity of a biological sample) in human blood using an external standard gene

In the description of this Example, a "target gene" refers to "target gene 1". Samples were prepared by taking 1.8 mL of pooled human blood having blood from multiple people mixed as a specimen and adding thereto 600, 2,000, 6,000, 20,000, 60,000, 200,000, 2,000,000, and 20,000,000 copies of a plasmid containing a target gene and used as calibration curve samples (standard solutions). Another samples were prepared by adding 2,000 copies of a plasmid containing the target gene to 1.8 mL of blood from each of eight donors and used as QC samples. To each of all these samples, 1,000 ng of dog genomic DNA (Zyagen) was added as an external standard gene. Genomic DNA was extracted from each of these samples using a commercially available DNA extraction reagent (QIAamp DNA Blood Midi Kits, QIAGEN), and then, qPCR quantification was performed using probes, forward primers and reverse primers for the target gene and the dog genomic DNA-specific gene (MC1R). The qPCR was performed using QuantStudio 7 Flex real-time PCR system (Thermo Fisher Scientific Inc.) for 40 cycles under conditions of 95°C for 10 min/(95°C for 15 seconds and 60°C for 1 minute) as one cycle.

A calibration curve was created using the difference (Delta Ct) between the Ct value of the target gene and the Ct value of the dog genomic DNA, and the concentration of the target gene added in each calibration curve sample (Figure 2A). The linearity of the calibration curve was observed in a copy number range for the target gene from 0.33 to 11,111 copies/µL blood, which suggests that the present method attains a quantitative measurement of the number of copies per unit quantity of the biological sample.

Using this calibration curve, the number of copies of the target gene in the QC samples prepared from the blood of each of the eight donors was calculated, and the results are shown in Figure 2D (right side). In every QC sample from the donors, the value (Observed) calculated with respect to the addition concentration (Nominal) had an accuracy of 87.2 to 114.9% and a CV (coefficient of variation) of 9.8%, which were good, indicating excellent quantitativity of the present method.

### [Comparative Example 2] Quantitative determination of a concentration of a target gene (the number of copies per unit quantity of a biological sample) in human blood using an internal standard gene

In the description of this Comparative Example, a "target gene" refers to "target gene 1". In quantitatively determining the number of copies of a gene using qPCR, it is common that the numbers of copies of a target gene among samples are corrected using an internal standard gene (by subtracting the Ct for the internal standard gene from the Ct for the target gene). Thus, RNaseP was selected as an internal standard gene to perform qPCR quantification on all of the DNA extracts of Example 2 using a probe, forward primer, and reverse primer for human RNaseP (TaqMan RNase P Control Reagents Kit, Thermo Fisher Scientific Inc.). The qPCR was performed using QuantStudio 7 Flex real-time PCR system (Thermo Fisher Scientific Inc.) for 40 cycles under conditions of 95°C for 20 seconds/(95°C for 1 second and 60°C for 20 seconds) as one cycle.

A calibration curve was created using the difference (Delta Ct) between the Ct value of the target gene and the Ct value of the RNaseP, and the concentration of each calibration curve sample (Figure 2B). Linearity of the calibration curve was observed in a copy number range for the target gene from 0.33 to 11,111 copies/µL blood.

On the other hand, the calculated number of copies of the target gene in the QC samples of each of the eight donors had an accuracy of 73.4 to 159.9% and a CV (coefficient of variation) of 26.7%, resulting in lower quantitativity compared to Example 2 (Figure 2D (left side)). The DNA concentration in the DNA extract was measured by absorption spectroscopy with 260 nm (Cytation 5, BioTek Instruments) and examined for correlation with accuracy; a significant negative correlation was then observed, which suggests that the relative concentration of the target gene to the internal standard gene fluctuates according to the differences in DNA concentration among the donors (Figure 2C). These results show the superiority of the quantitative determination using the external standard gene as shown in Example 2.

### [Example 3] Quantitative determination of a concentration of a target gene derived from genetically-engineered T cells (the number of copies per unit quantity of a biological sample) in human blood and a buffer solution

In the description of this Example and this Comparative Example, a "target gene" refers to "target gene 1". Samples were prepared by taking 0.5 mL of human blood, human blood with leukocytes removed by a commercially available leukocyte removal filter (PLASMODIPUR, EuroProxima), and buffer solution as specimens and adding thereto 4,000, 40,000, 400,000, and 4,000,000 genetically-engineered human T cells, respectively. The genetically-engineered human T cells described above were produced by introducing CAR gene including a target gene into T cells activated by a retroviral vector. As calibration curve samples (standard solutions), another samples were prepared by adding 375, 625, 1,250, 3,750, 12,500, 37,500, 125,000, 1,250,000, 12,500,000, and 125,000,000 copies of a plasmid containing the target gene to 0.5 mL of human blood. To each of all these samples, the dog genomic DNA (Zyagen) was added at 2,000 ng/mL. Genomic DNA was extracted from each of these samples using a commercially available DNA extraction reagent (QIAamp DNA Blood Midi Kits, QIAGEN), and then, qPCR quantification was performed using probes, forward primers and reverse primers for the target gene and the dog genomic DNA. The qPCR was performed using QuantStudio 7 Flex real-time PCR system (Thermo Fisher Scientific Inc.) for 40 cycles under conditions of 95°C for 10 minutes/(95°C for 15 seconds and 60°C for 1 minute) as one cycle.

A calibration curve was created using the difference (Delta Ct) between the Ct value of the target gene and the Ct value of the dog genomic DNA, and the concentration of the target gene added in each calibration curve sample, and the results of measuring the concentration of the target gene in the sample with the genetically-engineered T cells added are shown in Figure 3A. In every specimen, it was confirmed that the number of copies of the target gene increased in proportion to the number of cells added. In addition, the number of copies of the target gene derived by the present method was equivalent regardless of the type of specimen. These results suggest that the present method attains a quantitative measurement of the fluctuation in the number of copies of the target gene per unit quantity of a biological sample according to the increase or decrease of the genetically-engineered T cells *in vivo.*

### [Comparative Example 3] Quantitative determination of the number of copies of a target gene relative to the quantity of genomic DNA in a DNA extract

In the description of this Example and this Comparative Example, a "target gene" refers to "target gene 1". As a unit used for quantitative determination of a target gene in gene therapy, a proportion of the number of copies of the target gene to the quantity of genomic DNA is typically used. Accordingly, the human genomic DNA and the number of copies of the target gene in the DNA extracted from the sample with the genetically-engineered T cell added, prepared in Example 3, were measured to calculate the proportion of the number of copies of the target gene to the quantity of genomic DNA.

Samples were prepared by adding plasmids containing the target gene to extracts AE of the DNA extraction reagent at 1.5, 5, 15, 25, 50, 150, 500, 1,500, 5,000, 50,000, 500,000, and 5,000,000 copies/µL and used as calibration curve samples (standard solutions) for the target gene. In addition, another samples were prepared by adding human genomic DNA (Promega Corporation) to the AEs at 5.625, 56.25, 562.5, 5,625, 56,250, 112,500, and 225,000 pg/µL and used as calibration curve samples (standard solutions) for measuring the content of the human genomic DNA. qPCR quantification was performed on the DNA extracts of the calibration curve samples and the samples with the genetically-engineered T cell added as prepared in Example 3, using probes, forward primers, and reverse primers for the target gene and the human genomic DNA-specific gene sequence (RNaseP). For qPCR, QuantStudio 7 Flex real-time PCR system was used. The qPCR measurement on the target gene was performed for 40 cycles under conditions of 95°C for 10 minutes/(95°C for 15 seconds and 60°C for 1 minute) as one cycle. The qPCR measurement on the human genomic DNA was performed for 40 cycles under conditions of 95°C for 20 seconds/(95°C for 1 second and 60°C for 20 seconds) as one cycle.

A calibration curve was created using the Ct value of the target gene amplification curve at each concentration (the number of copies) of the calibration curve sample for the target gene and the concentration of each calibration curve sample, and the number of copies of the target gene in the DNA extract of the sample with the genetically-engineered T cells added was calculated. In addition, a calibration curve was created using the Ct value of the target gene amplification curve at each concentration (the quantity of the human genomic DNA) of the calibration curve sample for the human genomic DNA and the concentration of each calibration curve sample, and the quantity of the human genomic DNA in the DNA extract of the sample with the genetically-engineered T cells added was calculated. From these results, the proportion of the number of copies of the target gene to the quantity of the human genomic DNA in the DNA extract of the sample with the genetically-engineered T cells added was calculated and compared with the number of cells added; the results are shown in Figure 3B. In the sample with the genetically-engineered T cells added to human blood, the increase in the number of copies of the target gene according to the number of cells added was confirmed, whereas in the leukocyte-removed human blood and buffer solution samples, the increase in the number of copies of the target gene according to the number of cells added was not clearly observed. When genetically-engineered T cells are added, the human genomic DNA derived from the genetically-engineered T cells will increase with the increase in the number of copies of the target gene; this is deemed as the reason why the increase in the number of copies of the target gene according to the number of cells added was not observed in the leukocyte-removed human blood, in which human genomic DNA contents were significantly lower than that in the untreated human blood, and in the buffer solution. When the proportion of the number of copies of the target gene to the quantity of the genomic DNA is used as the unit for quantitative determination of the number of copies of the target gene as described above, the quantity of genome contained in the specimen may affect the fluctuation in the number of copies of the target gene in response to the increase or decrease of the genetically-engineered T cells *in vivo;* therefore, in order to quantitatively examine the fluctuation in the number of copies of the target gene in response to the increase or decrease of the genetically-engineered T cells *in vivo* independently of the type of specimen, it is considered more desirable to measure the number of copies per unit quantity of biological sample as shown in Example 2. In particular, when the quantity of genomic DNA in the blood fluctuates due to chemotherapy or the like, the present method is considered more effective.

### [Example 4] Quantitative determination of a target gene in human blood with correction using an external standard gene

In the description of this Example, a "target gene" refers to "target gene 1". Samples were prepared by taking pooled human blood that is a mixture of 0.5 mL blood sampled from multiple people as a specimen and adding plasmids containing a target gene thereto at concentrations of 0.75, 1.3, 2.5, 7.5, 25, 75, 250, 2,500, 25,000 and 250,000 copies/µL blood and used as calibration curve samples (standard solutions). Also, samples were prepared by adding plasmids containing the target gene to the same pooled human blood specimen at concentrations of 7.5, 200, and 200,000 copies/µL blood and used as QC samples. To each of all these samples, 1,000 ng of dog genomic DNA (Zyagen) was added as an external standard gene. Genomic DNA was extracted from each of these samples using a commercially available DNA extraction reagent (QIAamp DNA Blood Midi Kits, QIAGEN), and then, qPCR quantification was performed using probes, forward primers and reverse primers for the target gene and the dog genomic DNA-specific gene (MC1R). The qPCR was performed using QuantStudio 7 Flex real-time PCR system (Thermo Fisher Scientific Inc.) for 40 cycles under conditions of 95°C for 10 minutes/(95°C for 15 seconds and 60°C for 1 minute) as one cycle.

A calibration curve was created using the difference (Delta Ct) between the Ct value of the target gene and the Ct value of the dog genomic DNA, and the concentration of the target gene added in each calibration curve sample. The created calibration curve was used to measure the number of copies of the target gene in the QC samples and calculate the relative error (= 1 - accuracy) and CV. The number of copies of the target gene was measured three times, each on a different day. The results are shown in Table 2. Robustness of the quantitative determination method of the present invention, which uses the external standard gene for correction, can be confirmed.

**[Table 2]**

| Nominal concentration (copies/µL blood) | 1^{st} day | | | 2^{nd} day | | | 3^{rd} day | | |
|---|---|---|---|---|---|---|---|---|---|
| | Mean obserbed concentration (copies/µL blood) | Relative error (%) | Coefficient of variation (%) | Mean obserbed concentration (copies/µL blood) | Relative error (%) | Coefficient of variation (%) | Mean obserbed concentration (copies/µL blood\) | Relative error (%) | Coefficient of variation (%) |
| 200,000 | 218,066.2 | 9.0 | 5.4 | 196,127.5 | -1.9 | 6.9 | 190,866.7 | -4.6 | 9.0 |
| 2,000 | 1,895.1 | -5.2 | 10.3 | 1,803.6 | -9.8 | 7.4 | 1,852.4 | -7.4 | 8.1 |
| 7.5 | 8.1 | 7.4 | 7.0 | 8.2 | 9.4 | 4.4 | 7.3 | -2.7 | 5.7 |
| Slope | -3.35 | | | -3.39 | | | -3.33 | | |
| Intercept | 8.35 | | | 8.25 | | | 8.37 | | |
| PCR efficiency (%) | 98.8 | | | 97.2 | | | 99.5 | | |
| R² | 1.000 | | | 0.999 | | | 1.000 | | |

### [Example 5] Quantitative determination of a target gene in mouse blood with correction using an external standard gene

In the description of this Example, a "target gene" refers to "target gene 1". Samples were prepared by taking 30 µL of immunodeficient mouse blood as a specimen and adding plasmids containing a target gene thereto at 50, 200, 600, 2,000, 6,000, 20,000, 60,000, 200,000, and 2,500,000 copies/µL blood and used as calibration curve samples (standard solutions). Another samples were prepared by adding plasmids containing the target gene at 50, 100, 200, 600, 4,000, 40,000, 400,000, and 2,000,000 copies/µL blood and used as quality control samples (QC samples). To each of all these samples, 1,200 ng of dog genomic DNA (Zyagen) was added as an external standard gene. Genomic DNA was extracted from each of these samples using a commercially available DNA extraction reagent (QIAamp DNA Blood Midi Kits, QIAGEN), and then, qPCR quantification was performed using probes, forward primers and reverse primers respectively for the target gene and the dog genomic DNA-specific gene (MC1R). The qPCR was performed using QuantStudio 7 Flex real-time PCR system (Thermo Fisher Scientific Inc.) for 40 cycles under conditions of 95°C for 10 minutes/(95°C for 15 seconds and 60°C for 1 minute) as one cycle.

A calibration curve was created using the difference (Delta Ct) between the number of PCR cycles (Ct value) at the time when the target gene amplification curve with each concentration (the number of copies) of the calibration curve samples for the target gene crosses over with a threshold and the Ct value for the amplification curve of the dog genomic DNA in each sample, and the concentration of each calibration curve sample. The created calibration curve was used to measure the number of copies of the target gene in the QC samples and calculate the relative error (= 1 - accuracy) and CV. The number of copies of the target gene was measured three times, each on a different day. The results are shown in Table 3. It is suggested that the extraction efficiency of the gene is lower when mouse blood is used than when human blood is used (Example 4); however, even when such mouse blood is used (e.g., at the stage of a pre-clinical trial), the utility of the quantitative determination method of the present invention, which uses the external standard gene for correction, can be confirmed.

**[Table 3]**

| Nominal concentration (copies/µL blood) | 1^{st} day | | | 2^{nd} day | | | 3^{rd} day | | |
|---|---|---|---|---|---|---|---|---|---|
| | Mean obserbed concentration (copies/µL blood) | Relative error (%) | Coefficient of variation (%) | Mean obserbed concentration (copies/µL blood) | Relative error (%) | Coefficient of variation (%) | Mean obserbed concentration (copies/µL blood) | Relative error (%) | Coefficient of variation (%) |
| 2,000,000 | 1,832,000 | -8.4 | 8.8 | 1,834,000 | -8.3 | 3.7 | 1,746,000 | -12.7 | 4.8 |
| 400,000 | 360,600 | -9.8 | 4.9 | 308,000 | -23.0 | 12.4 | 332,000 | -17.0 | 7.9 |
| 40,000 | 35,240 | -11.9 | 6.1 | 32,080 | -19.8 | 7.5 | 34,540 | -13.6 | 2.4 |
| 4,000 | 4,086 | 2.2 | 4.0 | 3,712 | -7.2 | 6.4 | 3,510 | -12.2 | 3.3 |
| 600 | 665 | 10.8 | 8.4 | 573 | -4.6 | 10.4 | 633 | 5.6 | 7.2 |
| 200 | 233 | 16.5 | 3.8 | 210 | 4.8 | 10.3 | 184 | -8.0 | 10.2 |
| 100 | 117 | 16.8 | 13.5 | 112 | 12.1 | 18.1 | 108 | 8.2 | 10.3 |
| 50 | 60 | 20.3 | 16.9 | 53 | 6.6 | 19.8 | 52 | 3.6 | 17.5 |
| Slope | -3.49 | | | -3.44 | | | -3.44 | | |
| Intercept | 14.16 | | | 12.71 | | | 12.61 | | |
| PCR efficiency (%) | 93.4 | | | 95.3 | | | 95.2 | | |
| R² | 0.998 | | | 0.999 | | | 0.999 | | |

### [Example 6]

Genetically-engineered human T cells (hereinafter referred to as "first genetically-engineered human T cells") were produced by introducing the CAR gene including the target gene 1 into T cells activated by a retroviral vector, and genetically-engineered human T cells (hereinafter referred to as "second genetically-engineered human T cells") were produced by introducing the CAR gene including the target gene 2 into T cells activated by a retroviral vector.

The first genetically-engineered human T cells were administered intravenously to xenograft mice at a dose of 5 × 10⁶ CAR+ cells/animal or 10 × 10⁶ CAR+ cells/animal. Blood was sampled from the submandibular vein of the mice 1, 3, 7, 10, 14, 17, 21, and 28 days after the administration. In addition, the second genetically-engineered human T cells were administered intravenously to xenograft mice at a dose of 10 × 10⁶ CAR+ cells/animal. Blood was sampled from the submandibular vein of the mice 1, 4, 8, 11, 15, 22, and 29 days after the administration.

Using the blood samples sampled at each of the above time points (except for the last day), the numbers of copies of the target genes 1 and 2 in the blood samples were quantitatively determined by the same measurement method as in Example 2 and Comparative Example 2 based on the calibration curves created using the external standard gene (dog genomic DNA) and the internal standard gene (RNaseP), respectively, in the same manner as in Example 2 and Comparative Example 2. The results are shown in Figures 4A, B, C, and D. For the blood sample for the first genetically-engineered human T cells, saturation is observed when the number of copies of the target gene 1 is expressed in first copies/µg gDNA by the quantitative determination method using the internal standard gene (vertical axis), whereas saturation is not observed when it is expressed in copies/µL blood by the quantitative determination method using the external standard gene according to the present invention (horizontal axis). The same trend is observed for the blood sample for the second genetically-engineered human T cells (Figure 4B). In addition, the correlation between the number of the second genetically-engineered human T cells in the blood sample for the second genetically-engineered human T cells (horizontal axis, cells/µL blood, measured by flow cytometry) and the level of introduction of the target gene 2 (the number of copies, vertical axis) is more clearly shown when the number of copies is expressed per µL blood by the quantitative determination method using the external standard gene according to the present invention (Figure 4D) than when the number of copies is expressed per µg gDNA by the quantitative determination method using the internal standard gene (Figure 4C). The quantitative determination method according to the present invention is considered as a more suitable method for evaluating cell kinetics with less risk of underestimating the proliferation of CAR-T cells *in vivo.*

## Claims

1. A quantitative PCR method (qPCR method) for measuring a quantity of a target gene included in a human cell, the method comprising a step of:
adding, to a biological sample including the human cell, a predetermined quantity of exogenous DNA that does not cross over with the target gene as an external standard for correcting a measured quantity of the target gene.

2. The qPCR method according to claim 1,
wherein the qPCR method is a real-time PCR method, and
correction of the measured quantity of the target gene comprises obtaining a difference (ΔCt) between a Ct value measured for the target gene and a Ct value measured for the exogenous DNA and converting the ΔCt into a quantity of the target gene.

3. The qPCR method according to claim 1, wherein correction of the measured quantity of the target gene comprises reflecting a recovery percentage obtained by comparing a measured quantity of the exogenous DNA through the qPCR method with a quantity of the exogenous DNA added to the biological sample.

4. The qPCR method according to claim 1, wherein the qPCR method is a real-time PCR method, and the measured quantity of the target gene is represented by a number of copies per unit quantity of the biological sample.

5. The qPCR method according to claim 1, wherein the human is a human who has genetically-engineered T cells administered and/or suffers from a disease that causes a decrease in leukocytes or has undergone a therapy that causes a decrease in leukocytes.

6. A kit for qPCR comprising a predetermined quantity of exogenous DNA that does not cross over with a target gene, the exogenous DNA serving as an external standard for correcting a measured quantity of the target gene.


SEQUENCE LISTING

<110>  Takeda Pharmaceutical Company Limited

<120>  Quantitative PCR Method and Kit Used for the Method

<130>  PT38-9051WO

<150>  JP2019-178755
<151>  2019-09-30

<150>  JP2020-127716
<151>  2020-07-28

<160>  9

<170>  PatentIn version 3.5

<210>  1
<211>  30
<212>  DNA
<213>  Artificial Sequence

<220>
<223>  Probe for canine MC1R gene

<400>  1
gccttggctg cgcaggctgc tgtggtgcag                                        30


<210>  2
<211>  27
<212>  DNA
<213>  Artificial Sequence

<220>
<223>  Forward Primer for canine MC1R gene

<400>  2
cgcccatgta ttacttcatc tgttgcc                                           27


<210>  3
<211>  21
<212>  DNA
<213>  Artificial Sequence

<220>
<223>  Reverse Primer for canine MC1R gene

<400>  3
cacggcgatg gcgcccagga a                                                 21


<210>  4
<211>  17
<212>  DNA
<213>  Artificial Sequence

<220>
<223>  Probe for Target Gene 1

<400>  4

cgactacagg gcctact                                                    17


<210>  5
<211>  21
<212>  DNA
<213>  Artificial Sequence

<220>
<223>  Forward Primer for Target Gene 1

<400>  5
ggagctgagg tccctgagaa g                                               21


<210>  6
<211>  19
<212>  DNA
<213>  Artificial Sequence

<220>
<223>  Reverse Primer for Target Gene 1

<400>  6
cctggcccca gtagtcgaa                                                  19


<210>  7
<211>  16
<212>  DNA
<213>  Artificial Sequence

<220>
<223>  Probe for Target Gene 2

<400>  7
ctgaggagcg aggatg                                                     16


<210>  8
<211>  19
<212>  DNA
<213>  Artificial Sequence

<220>
<223>  Forward Primer for Target Gene 2

<400>  8
ccggagtgct gctgatcag                                                  19


<210>  9
<211>  21
<212>  DNA
<213>  Artificial Sequence

<220>
<223>  Reverse Primer for Target Gene 2

<400>  9
gctgtgccag atcatgcagt a                                               21